(19) Europäisches Patentamt
European Patent Office
Office européen des brevets

(11) Publication number: 0 309 421 B1

(12) # EUROPEAN PATENT SPECIFICATION

(45) Date of publication of patent specification: 19.11.92 (51) Int. Cl.5: C07C 309/18, A61K 31/205

(21) Application number: 88830373.2

(22) Date of filing: 19.09.88

(54) **O-alkanoyl derivatives of 3-amino-2-hydroxypropanesulfonic acid having anticonvulsivant activity and pharmaceutical compositions containing same for the therapeutical treatment of epilepsy.**

(30) Priority: 21.09.87 IT 4840487

(43) Date of publication of application: 29.03.89 Bulletin 89/13

(45) Publication of the grant of the patent: 19.11.92 Bulletin 92/47

(84) Designated Contracting States: AT BE CH DE ES FR GB GR LI LU NL SE

(56) References cited:
EP-A- 0 213 054
EP-A- 0 263 911
DE-A- 3 337 026
GB-A- 1 419 097
US-A- 4 246 194

(73) Proprietor: SIGMA TAU Industrie Farmaceutiche Riunite S.p.A.
Viale Shakespeare, 47
I-00144 Rome(IT)

(72) Inventor: Bagolini, Carlo Alberto
Via Monte Faraone, 45
I-00141 Roma(IT)
Inventor: Pacifici, Licia
Via Saffo, 10
I-00125 Roma(IT)
Inventor: Ouaresima, Emma Teresa
Via Cratete di Mallo, 36
I-00124 Roma(IT)

(74) Representative: Fassi, Aldo, Dr.
c/o Sigma-Tau Industrie Farmaceutiche
Riunite S.p.A. Via Pontina, Km. 30, 400
I-00040 Pomezia (Roma)(IT)

EP 0 309 421 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel 3-APS (3-aminopropanesulfonic acid) derivatives having anticonvulsivant activity and the pharmaceutical compositions containing same for the therapeutical treatment of epilepsy.

More specifically, the compounds of the present invention are O-alkanoyl derivatives of 3-amino-2-hydroxy propanesulfonic acid having general formula (I):

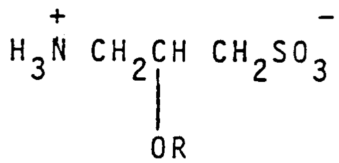

(I)

wherein R is alkanoyl having 2-5 carbon atoms and the pharmacologically acceptable salts thereof. Preferably, R is acetyl, propionyl or butyryl.

Clearly, the pharmacologically acceptable salts of the compound formula (I) have formula (Ia)

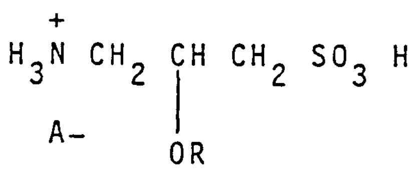

wherein $A^-$ is anion of the corresponding pharmacologically acceptable salt. For instance, $A^-$ is $Cl^-$.

The potent antiepileptic activity of 3-APS in some test models has long been known. 3-APS is structurally related to both GABA (gamma-aminobutyric acid) and taurine and possesses chemical electrophysiological and biochemical properties strikingly similar to GABA and GABA-agonists.

It has furthermore been shown that compounds capable of increasing GABA concentration in brain by blocking its enzyme degradation, possess antiepileptic activity. GABA itself has successfully been used in some epilepsy cases. However, neither GABA nor 3-APS easily cross the blood-brain barrier and consequently do not enter the central nervous system to any appreciable degree following oral or parenteral administration.

It has been found that the 3-APS derivatives of the present invention are capable of inducing an anticonvulsive effect more potent than that of 3-APS when they are administered via the usual oral or parenteral routes.

Compounds structurally similar to those of the present invention are known already. As the closest compound, L-3-amino-2-hydroxypropanesulfonic acid

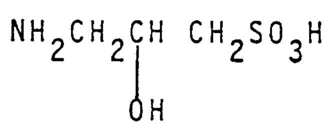

should be cited. This compound is a naturally-occuring substance and, in fact, it has been isolated from the red alga Polysiphonia fastigiata (Wickberg B., Acta Chem.Scand.11, 506 (1957). However, well before its isolation as a natural product, 3-amino-2-hydroxypropanesulfonic acid had been synthesized (Shigeru Tsunoo, Ber. 68, 1334-41,1935) from ammonia and 3-chloro-2-hydroxypropanesulfonic acid, that -in turn-had been obtained by reacting epichlorohydrin with sodium bisulfite.

As disclosed by Shigeru Tsunoo et al. in Jap. J. Pharmacol. 14, 393-400 (1964), thorough pharmacological searches were carried out in order to determine the activity of 3-amino-2-hydroxypropanesulfonic acid on the frog isolated heart as well as on the cardiac activity, blood pressure and breathing in dogs (Koizuka et al., Showa Med. J. 4, 91 (1942). Further searches were carried out with a view to studying the vasal action of this compound and its effects on adrenaline action (Tsuchida, Showa Med. J. 10, 280-290 (1950).

N-alkyl derivatives of the 3-amino-2-hydroxypropanesulfonic acid and salts thereof, e.g. with alkali metals, are also known.

Thus, for example, the British patent 1,419,097 to Procter & Gamble Ltd., discloses a process for producing quaternary ammonium compounds containing a long-chain lipophilic group, wherein an organic chloride of the general formula $R_1$ Cl in which $R_1$ is a primary alkyl radical from 8 to 20 carbon atoms is reacted in the presence of iodide ions, with a substituted tertiary amine of the general formula

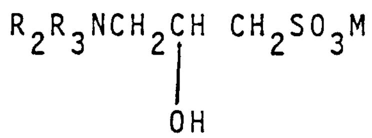

in which $R_2$ and $R_3$, which may be identical or different, are each an alkyl group having 1 to 6 carbon atoms and M is a metal cation, preferably sodium or potassium. The resulting quaternary ammonium compounds are useful as surfactants.

Further analogous compounds are e.g. disclosed in "Soap based detergent formulations: XII Alternate synthesis of surface active sulfobetaines", J.Am. Oil Chem.Soc. 53, n.2 60-63 (1976).

All such compounds are useful as surfactants in detergent compositions.

The relevant prior art can, therefore, be summarized as follows:

With regard to the compound which is the closest, from a structural viewpoint, to the compounds of the present invention, i.e. 3-amino-2-hydroxypropanesulfonic acid, its pharmacological properties in the cardiovascular field only have been examined, whilst its quaternary ammonium derivatives have been shown to possess utility as surfactants. The pharmacological properties on the central nervous system have not been investigated for anyone of these known compounds.

On the other hand, it does not appear that the O-esters of the 3-amino-2-hydroxypropanesulfonic acid of the present invention have ever been disclosed. Consequently, the compound that is the structurally closest one to the compounds of the present invention and whose anticonvulsivant properties have been disclosed is 3-APS. The activity of the compounds of the present invention is, however, unexpectedly superior to that of 3-APS.

The compounds of the present invention are prepared via known procedures: 3-amino-2-hydroxypropanesulfonic acid, prepared e.g. via the method disclosed in the above mentioned Ber. 68, 1334-1341 (1935), is subjected to Schotten-Bauman acylation. The overall reaction scheme is as follows:

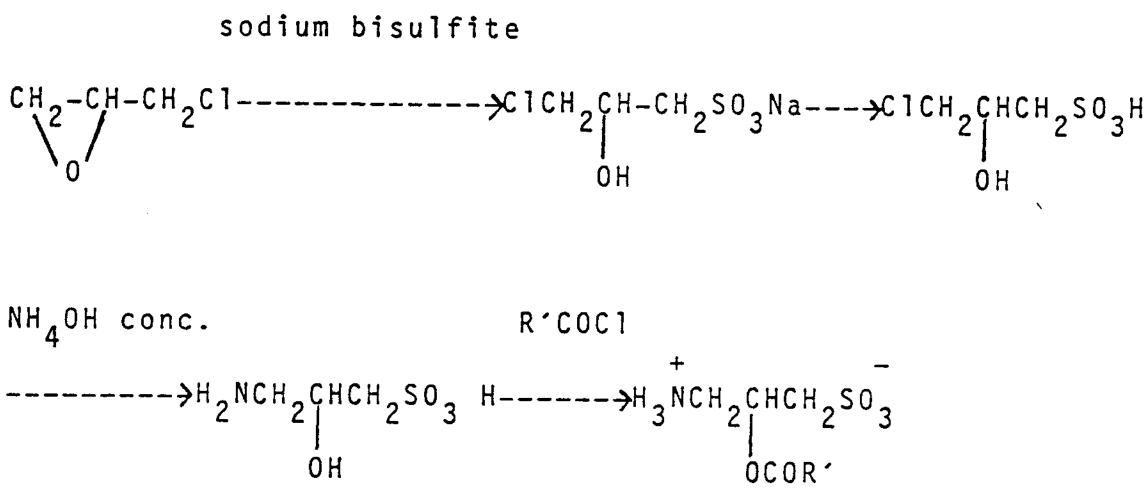


(R' = alkyl)

The following non-limiting examples illustrate the preparation of some of the compounds of the

invention.

Example 1

Preparation of 3-amino-2-acetyloxy propanesulfonic acid (ST549).

$$\overset{+}{H_3N}CH_2\underset{\displaystyle OCOCH_3}{\underset{|}{CH}}CH_2\,SO_3^-$$

13.5 g (0.146 moles) of epichlorohydrin were added to a solution of 15.26 g (0.08 moles) of sodium bisulfite in 45 milliliters of water. The reaction mixture was refluxed under stirring for 3 hours. Upon cooling first to room temperature and then to 0°C-5°C, a white solid (8 g) precipitated consisting of sodium 3-chloro-2-hydroxypropanesulfonate which was converted in the corresponding free acid with a strongly acidic resin (IR 120). Following concentration of the acid eluate, 7.5 g of 3-chloro-2-hydroxypropanesulfonic acid were obtained that were dissolved in 120 milliliters of conc.ammonia. The resulting solution was reacted at the reflux temperature overnight and then concentrated to dryness. The raw residue was purified with methanol. 5.3 g of 3-amino-2-hydroxypropanesulfonic acid were obtained.

These 5.3 g of acid, dissolved in 9 milliliters of trifluoroacetic acid, were reacted with 24 milliliters of acetyl chloride under stirring at 50°C overnight. The reaction mixture was poured dropwise in 400 milliliters of ethyl ether. A white precipitate formed that, following decantation of the ether phase, was taken up with methanol and again precipitated with ether. 3 g of 3-amino-2-acetyloxy propanesulfonic acid were thus obtained.

M.P.: 199-202°C

TLC ($CHCl_3$, MeOH, $NH_4OH$, $H_2O$, 55:35:5:5)

Elementary analysis

| | C | H | N | S |
|---|---|---|---|---|
| Calculated | 30.45% | 5.62% | 7.10% | 16.26% |
| Found | 30.01% | 5.85% | 7.20% | 16.40% |

NMR ($D_2O$) $\delta$=2.17 (3H, s,$CH_3CO$-); 3,23-3,47 (4H, m,-$NCH_2^-$ e - $CH_2S$-);

5.50 (1H, m, $-\underset{\displaystyle OCO-}{\underset{|}{CH}}-$)

Example 2

Preparation of 3-amino-2-butanoyloxypropanesulfonic acid hydrochloride (ST 477).

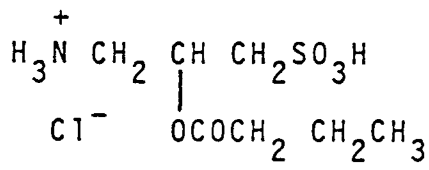

10.3 g (0.054 moles) of sodium bisulfite were added to a solution of epichlorohydrin (10 g; 0.108 moles) in 30 milliliters of water. The reaction mixture was refluxed under mechanical stirring for 3 hours. Upon cooling, a precipitate formed that was shown to be the sodium salt of 3-chloro-2-hydroxypropanesulfonic acid (8.8 g; yield: about 46%). The product was chromatographed on IRC 50 resin ($H^+$ form) by eluting with $H_2O$ (column diameter: 2.5 cm). The ratio 1:14 (g/ml) was maintained. 7 g of 3-chloro-2-hydroxypropanesulfonic acid, m.p. 250-251 ° C, were obtained.

A solution of 10 g (0.057 moles) of 3-chloro-2-hydroxypropanelsulfonic acid in 100 milliliters of conc. $NH_4OH$ was reacted under magnetic stirring at 100 ° C overnight. Following evaporation of the solution, the residue was crystallized from $H_2O$ -acetone, m.p.295-6 ° C.
The compound, dissolved in $H_2O$ and 6N HCl to convert it into its hydrochloride, was oven-dried in the presence of NaOH pellets and directly used in the subsequent reaction. 8.33 g (yield 76%) were obtained.

To a suspension of 2.0 g of hydrochloride in 40 milliliters of trifluoroacetic acid, 73.8 g (0.7 moles) of butyl chloride were added. The reaction mixture was heated up to 50 ° C and kept under magnetic stirring for 18 hours.

After it had been cooled to room temperature the mixtured was filtered and the filtrate added dropwise to an Erlenmeyer flask containing about 700 milliliters of ethyl ether. A white precipitate formed which was again dissolved in methanol, filtered and re-precipitated with ether. Upon drying 7.3 g of a white, hygroscopic solid product, stable at pH ≦6.3 were obtained; above this temperature deacylation of the compound takes place.

Elementary analysis:

| | C | H | N | Cl | S |
|---|---|---|---|---|---|
| Calculated | 32.12% | 6.16% | 5.35% | 13.54% | 12.25% |
| Found | 32.07% | 6.04% | 5.31% | 13.41% | 12.14% |

NMR corresponding

Example 3

Preparation of 3-amino-2-propionyloxy-1-propanesulfonic acid hydrochloride (ST 473).

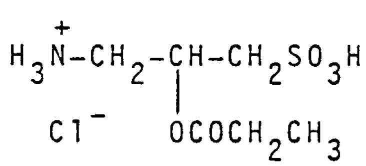

3-amino-2-hydroxypropanesulfonic acid (prepared as described in Example 1) (9.48 g; 0.038 moles) was dissolved in 11 milliliters of trifluoroacetic acid. Propionyl chloride (47 cc; 0.48 moles) was added to the resulting mixture. The mixture was kept under magnetic stirring at 50 ° C overnight. After cooling to room temperature the mixture was filtered. Ethyl eter was added to the filtrate till complete precipitation of a solid

product. The solid thus formed was filtered off and crystallized from acetone-ethyl ether. 2.7 g of a white hygroscopic compound were obtained. It was, therefore, impossible to measure its melting point.
TLC: silica gel

| Eluant: | $CHCl_3$ | $-MetOH-$ | $H_2O$ | $-NH_4OH$ |
|---|---|---|---|---|
| | 55 | 35 | 5 | 5 |

Rf: 0.7

NMR $D_2O$ 5,3(1H,m,-CH-);3,1-3,4 (4H,m, $N-CH_2$;$\underline{CH}_2SO_3H$);2,5(2H,
O
q, $\underline{CH}_2-CH_3$); 1,1 (3H,t,$-CH_3$ )

## PHARMACOLOGICAL TESTS

### Toxicity

The toxicity tests were carried out on male and female Sprague Dawley rats which were orally administered ST549 at the doses of 150-450-1350 mg/10 ml/kg for 15 consecutive days; the control animals were administered the same volume of vehicle as the test animals.

The compound did not affect either general health or behaviour of the animals. In all treated animals blood and urine parameters were unchanged with respect to the controls. Autopsy was performed at the end of the test and did not reveal either alterations or lesions to the main organs.

### Tolerability

The tolerability test was carried out on male albino swiss mice weighing 22-24 g. The compound ST 549, dissolved in sterile saline and intravenously administered at neutral pH, was well tolerated at the dose of 1.4 g/kg.

### Anticonvulsivant tests

Anticonvulsivant tests were carried out using cardiazol, strychnine and bicuculline at doses of 100 mg/10 ml/kg i.p., 0.73 mg/10 ml/kg s.c. and 2.38 mg/10 ml/kg s.c., respectively, chosen in such a way as to provoke death in 50% of the control animals.

The tests were carried out in male albino swiss (Charles River) mice weighing 22-24 g. The compound under examination, dissolved in water, was i.p. administered 30 minutes before the convulsivant agent administration.

As reference substance, 3-APS (200-400-800 mg/10 ml/kg i.p.) was used.

As activity parameters the following were considered: latency time of first convulsion, percentage of animals exhibiting convulsions, death latency time and death rate.

The test results relating to 3-APS are illustrated in Table 1. 3-APS at doses of 200,400 and 800 mg/10 ml/kg i.p. did not preserve animals from cardiazole-, strychnine-and bicuculline-induced death.

Table 1

3-APS activity on cardiazol-, strychnine- and bicuculline- induced convulsions.

| Compound 3-APS | Doses mg/kg i.p. | % Conv. | Cardiazol 1st conv. latency time (min.) | % Death rate | Death latency time (min.) | % Conv. | Strychnine 1st conv. latency time (min.) | % Death rate | Death latency time (min.) |
|---|---|---|---|---|---|---|---|---|---|
| $H_2O$ | 10 ml | 90 | 1'24"±0.31 | 50 | 20'00"±10,77 | 100 | 5'24"±0,69 | 100 | 8'06"±0.20 |
| 3-APS | 200 | 100 | 1'42"±0.26 | 50 | 4'24"±1,12 | 100 | 6'00"±0,53 | 90 | 6'43"±0.52 |
| $H_2O$ | 10 ml | 100 | 1'36"±0.40 | 70 | 19'17"±6,34 | 100 | 4'00"±0,21 | 100 | 4'54"±0,57 |
| 3-APS | 400 | 100 | •1'48"±0.70 | 90 | 9'53"±2,45 | 100 | 4'00"±0,39 | 100 | 6'23"±0,68 |
| $H_2O$ | 10 ml | 100 | 1'36"±0.40 | 70 | 19'17"±6,34 | 100 | 5'30"±0.45 | 80 | 9'38"±1,15 |
| 3-APS | 800 | 100 | 3'24"±0.98 | 60 | 15'40"±4.51 | 100 | 5'54"±0.50 | 90 | 10'27"±1,67 |

| Compound 3-APS | Doses mg/kg i.p. | % Conv. | Bicuculline 1st conv. latency time (min.) | % Death rate | Death latency time (min.) |
|---|---|---|---|---|---|
| $H_2O$ | 10 ml | 100 | 5'54"±0.43 | 80 | 6'53"±1,03 |
| 3-APS | 200 | 100 | 6'48"±0,39 | 100 | 7'30"±0.75 |
| $H_2O$ | 10 ml | 88* | 6'47"±0.60 | 75 | 6,33"±0.21 |
| 3-APS | 400 | 100* | 7'29"±0,56 | 75 | 7'40"±0.33△ |
| $H_2O$ | 10 ml | 80 | 6'53"±0,63 | 40 | 9'30"±2,78 |
| 3-APS | 800 | 70 | 9'08"±0,48■ | 40 | 9'30"±0,65 |

CONV. = CONVULSIONS.
10 animals per group (* = 8 animals per group ) were used.
Student's "t" test.■ and △ indicate P = 0.02 and P = 0.01 respectively with reference to the control group.
(' means minutes; " means seconds).

At 400 mg/10 ml/kg the latency time of bicuculline-induced death significantly increased, while at 800 mg/kg the latency time of the bicuculline-induced first convulsion significantly increased.

The test results relating to the compound under examination are illustrated in Table 2.

ST 549, at a dose equimolar to 400 mg/10 ml/kg i.p. of 3-APS, preserved 100% animals from cardiazol-induced death; the lower dose equimolar to 200 mg/10 ml/kg of 3-APS) did not affect the relevant parameters. At the doses equimolar to 400 and 200 mg/10 ml/kg i.p. of 3-APS, ST 549 decreased bicuculline-induced death rate by 30% and preserved from convulsions the same percentage of animals.

The lower dose significantly increased the latency time of strychnine-induced first convulsion and death.

Table 2

Activity of ST 549 administered i.p. on the c diazol; strychnine- and bicuculline- Juced convulsions.

| Compound | Doses mg/kg eq. 3APS | % Conv. | Cardiazol 1st conv. latency time (min.) | % Death rate | Death latency time (min.) | % Conv. | Strychnine 1st conv. latency time (min.) | % Death rate | Death latency time (min.) |
|---|---|---|---|---|---|---|---|---|---|
| $H_2O$ | 10 ml | 100 | 1'12"±0,13 | 50 | 15'24"±5,03 | 100 | 4'42"±0,21 | 100 | 5'18"±0,37 |
| ST 549 | 200 | 100 | 1'06"±0,10 | 50 | 20'36"±8,74 | 100 | 6'12"±0,63 * | 90 | 7'27"±0,87 * |
| ST 549 | 400 | 90 | 1'54"±0,69 | 0 | - | 100 | 6'06"±0,89 | 90 | 6'13"±1,06 |

| Compound | Doses mg/kg eq. 3APS | % Conv. | Bicuculline 1st conv. latency time (min.) | % Death rate | Death latency time (min.) |
|---|---|---|---|---|---|
| $H_2O$ | 10 ml | 100 | 6'30"±0,48 | 90 | 11'20"±1,78 |
| ST 549 | 200 | 70 | 7'26"±0,53 | 60 | 9'10"±1,67 |
| ST 549 | 400 | 60 | 7'00"±0,52 | 60 | 11'40"±0,76 |

Eq. = equimolar; CONV. = CONVULSIONS;
10 animals per group were used
Student's "t" test. * indicate = 0,05 with a reference to the control group.

Antiepileptic activity test ($FeCl_3$-induced epilepsy model).

Male albino Wistar rats weighing 250-300 g were used. The animals, anaesthetized with Nembutal Na

(30 mg/kg e.p.), were stereotactically injected in their left ventral hippocampus one microliter of 0.55 M $FeCl_3$ solution (AP =-3.2; L=5.0; H=5.5 Pellegrino and Cushman, 1971). Ten minutes following injection, recording electrodes were implanted: in both ventral hippocampi, two millimeters below the $FeCl_3$ injection site (bipolars), bilaterally on the frontoparietal cortex (monopolars). Recording of cerebral bioelectric activity, carried out with an EEG OTE-Biomedica PF 14e recorder, began about one hour following $FeCl_3$ injection (when the animal, after the anaesthetic effect had subsided, had regained the straightening reflex) and lasted more than three hours on end. The epilepsy degree was assessed by calculating for each animal the number and duration in seconds of the seizures at 5-minute intervals during the whole recording span both in the hippocampus where the injection had been performed and in the contralateral cortex. Moreover, an "index of epileptization" was calculated as the mean product of seizure number times the duration of the seizures in each time interval. 500, 250 and 125 mg/kg of the compound under examination, dissolved in sterile saline, were intravenously administered at neutral pH ninety minutes following $FeCl_3$ injection.

Three groups of control animals received, at the same time and via the same administration route as the test animals, an identical volume of vehicle.

The results thus obtained show the antiepileptic activity of the compound assessed by examining the whole phenomenon as it evolves in time via the measure (carried out with the trapezoidal rule) of the area under the curve wherein the average number of seizures and their duration is plotted versus time.

By comparing the areas relating to the controls with the areas relating to the treated animals via Mann-Whitney's "U" test, it was assessed that ST 549, at the tested doses, presented a statistically significant difference ($P<0.05$) with respect to the controls.

Auricular electroshock

Male albino Swiss mice weighing 22-24 g were used in this test. The electroshock conditions were the following:
shock duration: 3 seconds; frequence: 100; pulse amplitude: 0.6 milliseconds, current intensity: 40 milliamperes.

400 and 800 mg/10 ml/kg of the compound under examination were administered via the intraperitoneal route 30 minutes before the electroshock.

The tests results which illustrate the number of animals preserved from death provoked by maximal electroshock are shown in the following Table 3.

TABLE 3

Protective effect of ST 549 against auricular electroshock

| Compound | dose (mg/kg) | admin. route | number of animals | death rate |
|---|---|---|---|---|
| H20 | 10 ml/kg | i.p. | 14 | 6/14 |
| ST 549 | 400 | i.p. | 14 | 3/14 |
| ST 549 | 800 | i.p. | 14 | 3/14 |

Approximately 25-70 mg of a compound of general formula (I)/kg of body weight/day, was judges to be the appropriate dose for oral or parenteral administration.

However, lower or higher doses, based on sound physician's judment can be administered.

In practice, the compounds are orally or parenterally administered in any one of the usual pharmaceutical formulations which are prepared according to conventional procedures well-known to those skilled in pharmaceutical technology. These formulations comprise solid and liquid unit dosage forms, such as tablets, capsules and injectable forms, e.g. sterile solutions for ampules and vials.

## Claims

**1.** A compound having general formula (I)

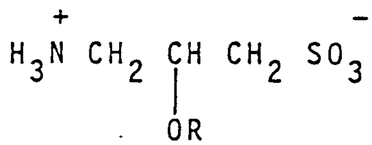

(I)

wherein R is alkanoyl having 2-5 carbon atoms,
and its pharmacologically acceptable salts.

**2.** The compound of claim 1, wherein R is acetyl, propionyl of butyryl.

**3.** As compound of claim 1, the compound

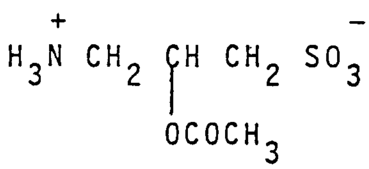

**4.** An orally or parenterally administrable pharmaceutical composition for treating epilepsy comprising an amount therapeutically effective for inducing an anticonvulsivant effect in an epileptic patient of a compound of general formula (I) or an equivalent amount of a pharmacologically acceptable salt thereof, and a pharmaceutically acceptable excipient.

**5.** The composition of claim 4, wherein the compound is the compound of claim 2.

**6.** The composition of claim 4, wherein the compound is the compound of claim 3.

## Patentansprüche

**1.** Verbindung der allgemeinen Formel (I)

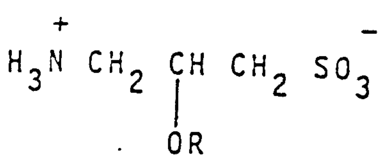

(I)

wobei R Alkanoyl mit 2 bis 5 Kohlenstoffatomen ist, und deren pharmakologisch geeignete Salze.

**2.** Verbindung nach Anspruch 1, wobei R Acetyl, Propionyl oder Butyryl ist.

**3.** Verbindung nach Anspruch 1, wobei die Verbindung

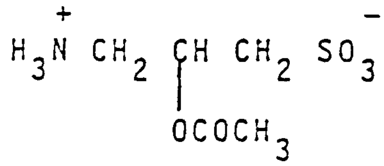

ist.

**4.** Oral oder parenteral verabreichbare pharmazeutische Zusammensetzung zum Behandeln von Epilepsie, umfassend eine therapeutisch wirksame Menge einer Verbindung der allgemeinen Formel (I) oder eine äquivalente Menge eines pharmakologisch geeigneten Salzes davon zum Induzieren einer krampfhemmenden Wirkung in einem Epilepsiepatienten und einen pharmazeutisch geeigneten Träger.

**5.** Zusammensetzung nach Anspruch 4, bei dem die Verbindung die Verbindung des Anspruchs 2 ist.

**6.** Zusammensetzung nach Anspruch 4, bei dem die Verbindung die Verbindung des Anspruchs 3 ist.

**Revendications**

**1.** Un composé répondant à la formule générale (I)

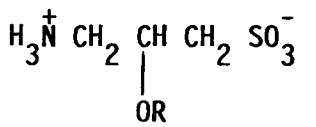
(I)

dans laquelle R est un alcanoyle ayant 2 à 5 atomes de carbone, et ses sels pharmacologiquement acceptables.

**2.** Le composé de la revendication 1, où R est un acétyle, propionyle ou butyryle.

**3.** Comme composé de la revendication 1, le composé

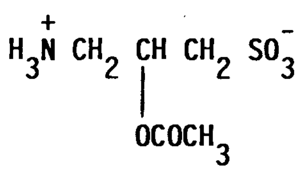

**4.** Une composition pharmaceutique administrable par voie orale ou parentérale pour le traitement de l'épilepsie, comprenant une quantité thérapeutique efficace pour provoquer un effet anticonvulsivant chez un patient épileptique d'un composé de formule générale (I) ou une quantité équivalente d'un sel pharmacologiquement acceptable de celui-ci, et un excipient pharmaceutiquement acceptable.

**5.** La composition de la revendication 4, dans laquelle le composé est le composé de la revendication 2.

**6.** La composition de la revendication 4, dans laquelle le composé est le composé de la revendication 3.